# EUROPEAN PATENT SPECIFICATION

(11) **EP 1 707 232 B1**
(45) Date of publication and mention of the grant of the patent: **04.08.2010**
(21) Application number: 06075629.3
(22) Date of filing: 10.03.2000
(51) Int. Cl.: A61M 15/00

(54) **Improvements relating to an inhalation device**
Verbesserungen an einem Inhalationsgerät
Améliorations apportées à un dispositif d'inhalation

(30) Priority: 10.03.1999 GB 9905538
(43) Date of publication of application: 04.10.2006
(62) Divisional of application: 00909505.0
(73) Proprietor: GLAXO GROUP LIMITED, Greenford, Middlesex UB6 0NN (GB)
(72) Inventor: Davies, Michael Birsha, Ware, Hertfordshire SG12 0DP (GB)
(74) Representative: Rice, Jason Neale

(56) References cited:
- WO-A-98/34663
- US-A- 5 201 308

## Description

The present invention relates to a device for the protection of a dose of medicament in an inhalation device for use in the administration of medicament to a patient.

The use of inhalation devices in the administration of medicaments, for example in bronchodilation therapy, is well known. Such devices generally comprise a body or housing within which a medicament container is located. A mouthpiece (or nozzle) is typically provided, wherein 'in use' the mouthpiece communicates with the medicament container to allow passage of medicament from the source to the mouthpiece and thence, to the patient.

In a typical dispensing operation the body of the device is held by the patient and the mouthpiece (or nozzle) of the inhalation device is placed in the mouth (or nose) of the patient. The patient inhales, thereby causing transfer of medicament from the medicament container to the interior of the body of the patient.

Many inhalers are known where the medicament is stored in a pocket, which is sealed for the purpose of preventing any loss of medicament during transportation or in order to reduce the moisture contamination of the medicament during the life of the inhaler. Examples of such are disclosed in EP0211595, where the medicament is loaded directly into a blister pack comprising a sheet; which may be laminated, of foil or plastics material which acts as a carrier and which is provided with a number of breakable or openable containers called "blisters" incorporating a sheet secured on a first sheet forming a cover or lid. A plunger can then be carried by the lid and arranged to penetrate a container when the lid is moved to its open position. A device disclosed in WO97/25086, comprises a spider having pads, resiliently urged into contact with the pockets of the device, said pads being raised away from the pocket by rotation of the spider prior to the use of the inhaler.

A device disclosed in US5,860,419 comprises medicament metered into blisters. The foil seal is peeled back to expose the medicament such that as the blister approaches the airway it is exposed for use.

However, the problem with these inhalers is that once they are prepared for use, i.e. once the seal of the pocket or "blister" is broken or removed, the medicament is exposed to the atmosphere inside the inhaler. The medicament could be dislodged from the pocket if the inhaler is shaken, dropped, or the user exhales into the device.

Similar problems exist with reservoir inhalers where the dose is metered in use. Examples of reservoir devices include that known as Turbohaler as described in EP0237507 and EP0069715, and the device described in WO97/20589.

Other inhalers are disclosed in WO98/34663 and US-A-5201308

Thus according to the present invention there is provided a dry powder inhaler according to claim 1 herein.

Amongst the advantages of the present invention in one or more of its embodiments are included that it provides a reduction in moisture contamination of the medicament, particulate contamination of the dose, loss of the dose if the device is inverted, dropped or shaken, until the point of inhalation by the patient. Air exhaled into the device by the patient does not come into contact with the medicament, thereby reducing contamination of the medicament by moisture or particulates, or the medicament being dislodged from the pocket by the patient exhaling into the device.

It follows that the advantages of the present invention include that it also ensures that the full dose metered is available to the patient in that part of the dose is prevented from being lost such as, for example being lost within the device, if the complete dose is not taken in the first inhalation, as may be the case with the elderly and children.

The covering means is only open in the presence of airflow through the airway in a first direction after which it preferably returns to its covering position.

The airflow through the airway in the first direction will generally be caused by patient inhalation.

The covering means is preferably in biased contact with the dose or container retaining the dose. Alternatively the covering means senses airflow through the airway and responds thereto. Thus the covering means responds to airflow through the airway in a first direction by opening.

The means for sensing the airflow through the airway and/or the means of responding thereto (i.e. opening said covering means and/or covering the dose more effectively) may incorporate electronic means. Alternatively it may incorporate mechanical or electromechanical means.

When airflow through the airway is in the second direction the covering means preferably responds by more effectively protecting the dose. For example it may more effectively protect the dose by being urged into closer contact with the dose or container retaining the dose.

The airflow in the second direction may generally be caused by the patient exhaling into the device.

The covering means may comprise one or more poppet valves, diaphragm valves, rotary valves, reciprocating valves, sealing flaps or a combination thereof. Preferably, the covering means is a sealing flap.

There are various ways in which a dose can be metered. In one manner the metering occurs during the manufacturing process of the device ("pre-metered") and the metered dose is retained or located in discrete units. Alternatively the device contains a reservoir containing medicament and the dose is metered in use.

Metering may be based on volume of medicament or surface area.

For metering based on volume the dose may be metered in use or pre-metered into a container of the required volume. More specifically the container may be a pocket wherein the dose is retained after it has been metered. The pocket may also form a throughhole during operation of the device.

In the dose protector the dose or container retaining the dose may have a surrounding rim. The rim around the dose, container retaining the dose or pocket is not specifically to retain the dose but preferably acts to create a better contact with the said covering means.

Especially preferred is an inhaler in which the dose container is a pocket and the covering means is at least one sealing flap in biased contact with said pocket and providing a cover for the pocket; wherein the contact between the at least one sealing flap and the pocket is broken by airflow through the airway in the first direction, but not in the second opposite direction

Preferably, the sealing flap is spaced away from the pocket by the airflow once the contact with the pocket is broken.

Preferably the sealing flap is made of a flexible, resilient material with a memory, preferably a 3 year memory more preferably a greater than 3 year memory, i.e. the sealing flap stays resilient, biased against the pocket and flexible.

Preferably the sealing flap vibrates in the airflow once the contact with the pocket is broken. This has the effect of increasing the fine particle mass. Features which may influence the ability of the sealing flap are the flexibility of the material, preferably the sealing flap is made of highly flexible material for example a thermoset rubber, to allow the vibration to occur as the pressure changes in the device; the resilience of the sealing flap, if the resilience is too high the sealing flap may not open, if too low the sealing flap may not close therefore vibrations would not occur.

Preferably the sealing flap is of equivalent or slightly reduced width relative to the distance between the inside walls of the housing at the base of the walls of the housing where the sealing flap is in contact with the pocket. Preferably the distance between the inside walls of the housing increases as the distance away from the pocket increases preventing the sealing flap being hampered in its movement as it vibrates.

It will be appreciated that the vibrations of the sealing flap can be affected by the length of the sealing flap and its anchor position on the housing relative to the position of the pocket. The height of the airway may also effect the vibration of the sealing flap by influencing the curve of the sealing flap. The curve of the sealing flap can be affected by having a locally thinned area on the sealing flap which acts as a hinge, instead of or as well as being made of a highly flexible material.

The covering means is preferably spaced away from the dose or container retaining the dose to coincide with the airflow through the airway in the first direction once the contact with the dose or container retaining the dose is broken.

In alternative embodiments of the invention the covering means may vibrate in the airflow through the airway in the first direction.

The housing preferably contains a valve flap such that when the airflow is in a second opposite direction, the airflow exits the housing by means of the valve flap. This arrangement allows the airflow to escape from the housing, to prevent build up of pressure.

The said covering means preferably protects the dose from the patient exhaling into the device, moisture contamination, particulate contamination, loss of the dose (e.g. in the device) or a combination thereof.

Preferably the dose protector additionally comprises a fixed seal. This provides a further means by which to prevent loss of drug that can occur in transportation. It also can aid in reducing moisture contamination of the medicament during storage and transportation of the device. This seal (e.g. a pocket seal) will need to be removed before the patient uses the device, but protection of the dose after removal of the pocket seal will still be maintained by the covering means (e.g. sealing flap).

The inhaler may comprise a body, and a mouthpiece.

Preferably, the contact between the at least one sealing flap and the pocket is broken by airflow towards the mouthpiece.

Even more preferably the contact between the at least one sealing flap and the pocket is not broken by airflow from the mouthpiece through the body.

An embodiment of the inhalation device according to the present invention will now be described with reference to the accompanying drawings in which:
Fig. 1a is a sectional side view of a dose protector wherein the sealing flap is at rest.
Fig. 1b is a sectional side view of a dose protector wherein the airflow is in the direction to activate the sealing flap.
Fig. 2a is a sectional side view of a device as shown in Fig 1 a and Fig 1b, wherein the valve flap is at rest.
Fig. 2b is a sectional side view of a device as shown in Fig 1a and Fig 1b, wherein the valve is open.
Fig. 2c is a sectional side view of a device as shown in Fig 1 a and Fig 1b wherein the airflow is in the direction to activate the sealing flap.
Fig. 3a is a sectional side view of a device as shown in Fig 1a and Fig 1b according to the present invention, wherein the pocket has geometry particularly suitable for a metered dose inhaler.
Fig. 3b is a sectional side view of a device as shown in Fig 1a and Fig 1b, wherein the airflow is in the direction to activate the sealing flap.
Fig. 4a is a dose protector with closure mechanism in the closed position.
Fig. 4b is a dose protector with the closure mechanism in the open position.
Fig. 5 is a sectional side view of a device as shown in Fig 1a and Fig 1, with a pocket seal.
Fig. 6 is a sectional side view of a unit dose powder inhaler having a dose protector as shown in Fig 1a and Fig 1b, where the sealing flap is at rest.
Fig. 7 is a perspective view of a unit dose inhaler having a dose protector shown in Fig 1a and Fig1b at rest.
Fig. 8 is a perspective view of a moulding for a unit dose inhaler similar to that shown in Fig. 6.
Fig 9 is a different perspective view of a unit dose inhaler similar to that shown in Fig. 6

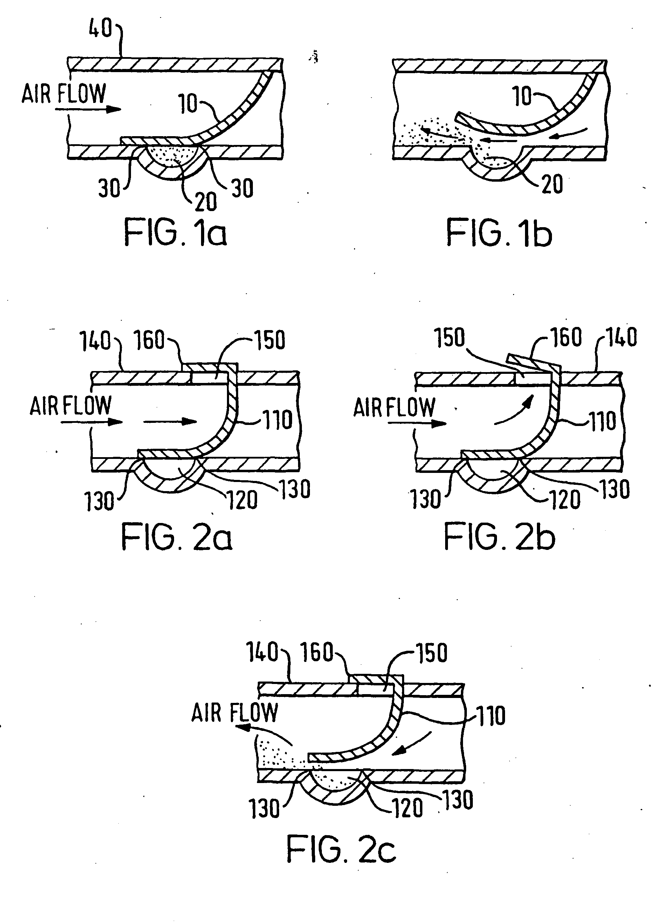

Figures 1a and 1b show a first dose protector comprising a sealing flap 10 and a pocket 20 suitable for containing a dose of medicament. The sealing flap 10 is resiliently biased into contact with the edge of the pocket 30 to reduce moisture contamination and loss of medicament during transportation. The sealing flap is fixedly attached to the body of the housing 40. When air flows towards the sealing flap as shown in Fig. 1b, such airflow being created by inhalation by the patient, the contact between flap 10 and the edge of the pocket 30 will break, and medicament is entrained in the airflow.

The sealing flap can vibrate to increase the fine particle mass of the medicament.

Fig. 1a shows that when the air flows towards the sealing flap 10 in the direction shown, the contact between the sealing flap 10 and the edge of the pocket 30 is not broken, thereby maintaining the protection of the dose from moisture and particulate contamination. Such airflow would usually be created by exhalation.

Figures 2a, 2b and 2c show a dose protector having a hole 150 in the housing 140 so when the airflow is due to exhalation by the patient the valve flap 160 opens to let the moist air out. However when the airflow is in the opposite direction the contact between the sealing flap 110 and the edge of the pocket 130 is broken.

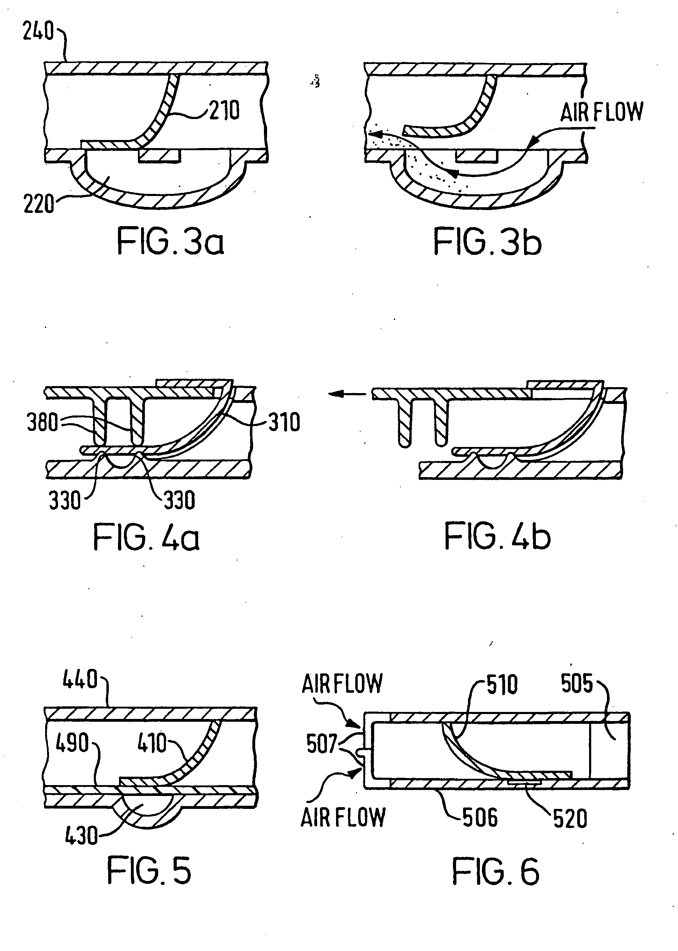

Figures 3a and 3b show a dose protector according to the invention having a sealing flap 210 and a pocket 220 having geometry particularly suitable for a metered dose inhaler.

Figures 4a and 4b show the closure mechanism in an open and closed position. The locking means having slidable movement relative to the pocket where in the closed position the legs 380 sit over the edge of the pocket 330 trapping the sealing flap 310 between the legs and the edge of the pocket 330 preventing movement of the sealing flap 310. To open the device, the closure mechanism is slid relative to the pocket so that the legs 380 are not over the rim of the pocket and the sealing flap is not trapped, but free to move when airflow is in the correct direction.

Fig. 5 shows a dose protector additionally having a pocket seal 490, which may be laminated, of foil or plastics material, which is removed to ready the device for use by the patient.

Fig. 6 shows a unit dose powder inhaler having a body 506 a mouthpiece 505 air inlet holes 507 a sealing flap 510 and a pocket 520. When the patient inhales through the mouthpiece 505 air flows into the device through the air inlet holes 507 and the contact between the sealing flap 510 and the pocket 520 is broken. The medicament present in the pocket 520 is entrained in the airflow and carried through the device to be administered to the patient.

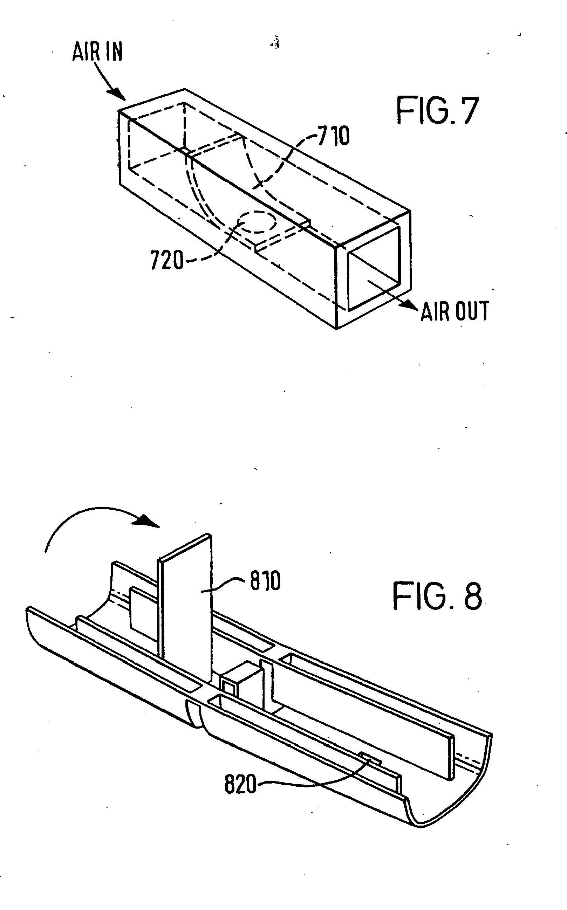

Fig 7 shows a unit dose inhaler wherein pocket 720 containing medicament is covered by sealing flap 710.

Fig 8 shows a one-piece moulding for a unit dose powder inhaler similar to that illustrated in Fig. 6 wherein 820 is the pocket and 810 is a sealing flap which bends into position when one half of the moulding is folded over and closed to form the finished device.

Mouldings as shown in Fig. 8 may be formed in arrays which allows for convenient filling of the pockets with medicament in an automated process.

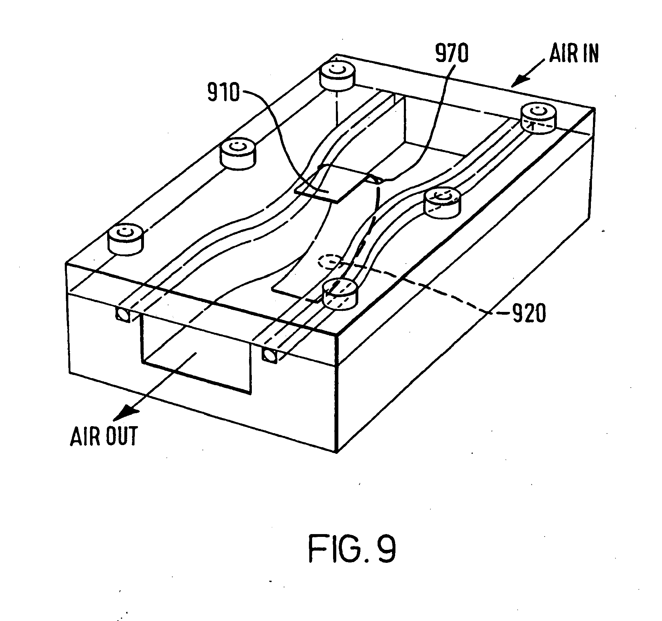

Fig. 9 shows a unit dose inhaler with a pocket containing medicament 920 and a flexible resilient sealing flap 910 located fixedly through a tight fitting slot 970 in the top of the device. The material of the sealing flap is thinned at the slot to improve its positioning.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

## Claims

1. A dry powder inhaler comprising a housing (240) defining an airway, a dose of medicament powder retained in a dose container (220), and a dose protector comprising covering means (210) for said dose, the airway is in fluid communication with the dose container through first and second openings to the dose container, the first opening being spaced from the second opening in a first direction, wherein the covering means is movable from a covering position in which it covers the first opening, but not the second opening, to an open position in which it opens the first opening, **characterised in that** the covering means is adapted so that it is only movable from the covering position to the open position in response to airflow through the airway in the first direction but not in a second, opposite direction whereby airflow through the airway in the first direction, but not the second, opposite direction, is able to pass through the dose container via the openings to entrain the medicament powder.

2. An inhaler as claimed in claim 1 wherein the covering means is biased to its covering position.

3. An inhaler as claimed in claim 1 or claim 2 wherein the airflow through the airway in the first direction is causable by patient inhalation.

4. An inhaler as claimed in any of claims 1 to 3 wherein the covering means is adapted to sense airflow through the airway in the first direction and respond thereto.

5. An inhaler as claimed in any of claims 1 to 4 where the covering means is a sealing flap.

6. An inhaler as claimed in any of claims 1 to 5 wherein the dose container is a pocket.

7. An inhaler as claimed in claim 5 wherein the sealing flap is in biased contact with the first opening.

8. An inhaler as claimed in claim 5 or 7 wherein the sealing flap is made of thermoset rubber.

9. An inhaler as claimed in any one of claims 1 to 8 wherein the covering means is adapted in use to vibrate in the airflow through the airway in the first direction.

10. An inhaler as claimed in any preceding claim wherein the covering means protects the dose from the patient exhaling into the device, moisture contamination, particulate contamination and loss of the dose or a combination thereof.

11. An inhaler as claimed in any preceding claim further comprising a body and a mouthpiece.

## Patentansprüche

1. Trockenpulver-Inhalator, mit einem Gehäuse (240), das einen Luftweg definiert, einer Medikamentenpulverdosis, die in einem Dosis-Behälter (220) enthalten ist, und einem Dosis-Schutz, der eine Abdeckeinrichtung (210) für die Dosis umfasst, wobei der Luftweg mit dem Dosis-Behälter in FluidVerbindung ist durch eine erste und eine zweite Öffnung an dem Dosis-Behälter, die erste Öffnung von der zweiten Öffnung in einer ersten Richtung beabstandet ist, wobei die Abdeckeinrichtung aus einer Abdeckposition, in der sie die erste Öffnung bedeckt, aber nicht die zweite Öffnung, in eine offene Position bewegbar ist, in der sie die erste Öffnung öffnet, **dadurch gekennzeichnet, dass** die Abdeckeinrichtung so angepasst ist, dass sie lediglich aus der Abdeckposition in die offene Position als Reaktion auf einen Luftfluss durch den Luftweg in die erste Richtung, aber nicht in eine zweite, entgegengesetzte Richtung bewegbar ist, wodurch ein Luftfluss durch den Luftweg in die erste Richtung, aber nicht die zweite, entgegengesetzte Richtung imstande ist durch den Dosis-Behälter mittels der Öffnungen durchzugehen, um das Medikamentenpulver mitzureißen.

2. Inhalator nach Anspruch 1, wobei die Abdeckeinrichtung in ihre Abdeckposition vorgespannt ist.

3. Inhalator nach Anspruch 1 oder Anspruch 2, wobei der Luftfluss durch den Luftweg in die erste Richtung durch Patienteninhalation herbeiführbar ist.

4. Inhalator nach einem der Ansprüche 1 bis 3, wobei die Abdeckeinrichtung geeignet ist, einen Luftfluss durch den Luftweg in die erste Richtung wahrzunehmen und darauf zu reagieren.

5. Inhalator nach einem der Ansprüche 1 bis 4, wobei die Abdeckeinrichtung eine Dichtungsklappe ist.

6. Inhalator nach einem der Ansprüche 1 bis 5, wobei der Dosis-Behälter eine Tasche ist.

7. Inhalator nach Anspruch 5, wobei die Dichtungsklappe in vorgespanntem Kontakt mit der ersten Öffnung ist.

8. Inhalator nach Anspruch 5 oder 7, wobei die Dichtungsklappe aus Duroplast-Gummi hergestellt ist.

9. Inhalator nach einem der Ansprüche 1 bis 8, wobei die Abdeckeinrichtung geeignet ist, bei der Verwendung, in dem Luftfluss durch den Luftweg in die erste Richtung zu vibrieren.

10. Inhalator nach einem der vorhergehenden Ansprüche, wobei die Abdeckeinrichtung die Dosis vor dem Patienten, der in die Vorrichtung ausatmet, Feuchtigkeitskontamination, Partikel-Kontamination und Verlust der Dosis, oder einer Kombination davon, schützt.

11. Inhalator nach einem der vorhergehenden Ansprüche, ferner umfassend einen Körper und ein Mundstück.

## Revendications

1. Inhalateur de poudre sèche qui comprend un logement (240) définissant un conduit d'air, une dose de poudre de médicament contenue dans un récipient pour dose (220), et un protecteur de dose qui comprend un moyen de recouvrement (210) pour ladite dose, le conduit d'air se trouve en communication fluidique avec le récipient pour dose via des première et seconde ouvertures dans le récipient pour dose, la première ouverture étant espacée de la seconde ouverture dans une première direction, le moyen de recouvrement étant amovible d'une position de recouvrement dans laquelle il couvre la première ouverture, mais pas la seconde ouverture, à une position ouverte dans laquelle il ouvre la première ouverture, **caractérisé en ce que** le moyen de recouvrement est adapté pour n'être amovible qu'entre la position de recouvrement et la position d'ouverture en réponse à l'écoulement d'air dans le conduit d'air dans la première direction, mais pas dans une seconde direction opposée, l'écoulement d'air dans le conduit d'air dans la première direction, mais pas dans la seconde direction opposée, pouvant traverser le récipient pour dose via les ouvertures pour entraîner la poudre de médicament.

2. Inhalateur selon la revendication 1, dans lequel le moyen de recouvrement est dévié dans sa position de recouvrement.

3. Inhalateur selon la revendication 1 ou la revendication 2, dans lequel l'écoulement d'air dans le conduit d'air dans la première direction est entraîné par l'inhalation par le patient.

4. Inhalateur selon l'une quelconque des revendications 1 à 3, dans lequel le moyen de recouvrement est adapté pour détecter un écoulement d'air dans le conduit d'air dans la première direction et pour réagir à celui-ci.

5. Inhalateur selon l'une quelconque des revendications 1 à 4, dans lequel le moyen de recouvrement est une patte de fermeture.

6. Inhalateur selon l'une quelconque des revendications 1 à 5, dans lequel le récipient pour dose est une poche.

7. Inhalateur selon la revendication 5, dans lequel la patte de fermeture est en contact dévié avec la première ouverture.

8. Inhalateur selon la revendication 5 ou 7, dans lequel la patte de fermeture est formée par un caoutchouc thermodurcissable.

9. Inhalateur selon l'une quelconque des revendications 1 à 8, dans lequel le moyen de recouvrement est adapté durant l'utilisation pour vibrer dans l'écoulement d'air dans le conduit d'air dans la première direction.

10. Inhalateur selon l'une quelconque des revendications précédentes, dans lequel le moyen de recouvrement protège la dose de l'expiration du patient dans le dispositif, de la contamination par l'humidité, de la contamination particulaire et de la perte de la dose ou d'une combinaison de celles-ci.

11. Inhalateur selon l'une quelconque des revendications précédentes, qui comprend en outre un corps et un embout buccal.
